# EUROPEAN PATENT SPECIFICATION

(11) **EP 2 898 069 B1**
(45) Date of publication and mention of the grant of the patent: **11.04.2018**
(21) Application number: 13773470.3
(22) Date of filing: 18.09.2013
(51) Int. Cl.: C12N 15/10, C12Q 1/68

(54) **USE OF DIVALENT IONS, PROTEASES, DETERGENTS, AND LOW PH IN THE EXTRACTION OF NUCLEIC ACIDS**
VERWENDUNG VON DIVALENTEN IONEN, PROTEASEN, DETERGENZIEN UND NIEDRIGEN PH-WERTEN BEI DER EXTRAKTION VON NUKLEINSÄUREN
UTILISATION D'IONS DIVALENTS, DE PROTÉASES ET DE DÉTERGENTS SOUS UN FAIBLE PH POUR L'EXTRACTION D'ACIDES NUCLÉIQUES

(30) Priority: 19.09.2012 US 201261703112 P
(43) Date of publication of application: 29.07.2015
(73) Proprietor: Beckman Coulter, Inc., Brea, CA 92821 (US)
(72) Inventor: SRINIVASAN, Viswanathan, Westford, Massachusetts 01886 (US); CULLIS, Donald, Bedford, Massachusetts 01730 (US); LUU, Huyet, Dorchester, Massachusetts 02124 (US); RODRIGUEZ, Jorge A., Chino, California 91710 (US)
(74) Representative: Boult Wade Tennant
(86) International application number: PCT/US2013/060349
(87) International publication number: WO 2014/047141

(56) References cited:
- EP-A1- 1 911 844
- WO-A1-01/21830
- WO-A1-2011/057184
- US-A1- 2011 172 408

## Description

### BACKGROUND OF THE INVENTION

PCR-based diagnostic assays are a powerful tool for nucleic acid analysis, enabling the detection of even a single copy of a target nucleic acid molecule. Target nucleic acid sequences can be amplified by PCR and the amplified product can be detected and quantified.

The sensitivity and reliability of nucleic acid-based diagnostic assays depends on efficient unbiased methods of extracting pure, high-quality nucleic acids from biological samples. Nucleases that degrade nucleic acids and substances that inhibit the amplification process are present in biological samples. For example, DNase or RNase degrade target nucleic acids, EDTA chelates divalent cations such as Mg²⁺ that are essential for protease and nuclease activity and substances like heparin, phenol, denatured albumin, polyamines, polysaccharides and calcium alginate inhibit PCR.

Two major challenges in purification of nucleic acids, especially from clinical samples, are lysis and non-specific nuclease activity. In fact, during the purification process both extraction of nucleic acids and their degradation occur simultaneously. To overcome non-specific nucleic acid degradation, various approaches have been attempted. The most widely used techniques are the use of non-specific proteases and various ionic and non-ionic detergents to ensure complete lysis. However, for an optimal performance of the protease, a physiological pH range is required. At near physiological pH, various nucleases are active, as well, thus leading to loss of target nucleic acids.

EP1911844 A1 relates to methods for isolating a target nucleic acid from a sample and companion kits and reagents.

US 2011/172408 A1 relates to a method of separating nucleic acids using a solid phase capable of binding nucleic acids which is enhanced in the presence of an ethyleneamine compound.

WO 2011/057184 A1 relates to method for preserving and processing nucleic acids located within a blood sample.

WO 01/21830 A1 relates to methods and compositions for inhibiting and/or inactivating nucleases by employing nuclease inhibitors.

Effective nucleic acid extraction methods should minimize the activity of nucleases, thereby maintaining the integrity of the nucleic acids that are analyzed in the diagnostic assay. Similarly, inhibitors of amplification present in the sample are desirably reduced in amplification methods. The present invention provides these and other advantages.

### BRIEF SUMMARY OF THE INVENTION

The present invention is defined in the claims. In one aspect, the invention provides methods and compositions for extracting nucleic acids from biological samples by using divalent ions, proteases, detergents, and low pH conditions.

In one aspect, the present invention provides methods for extraction of target nucleic acid from a biological sample comprising cells or microorganisms, the method comprising:
(a) adding a transition metal salt, an alkaline earth metal salt, or combination thereof to the biological sample in an amount sufficient to inactivate nucleases in the sample;
(b) adding an extraction solution to the biological sample in an amount sufficient to lyse the cells or microorganisms, thereby forming a lysate;
(c) adding an acidic buffer to the lysate to an amount sufficient to reduce the pH of the lysate to between pH 1 to pH 5; and
(d) separating the target nucleic acid from the lysate to extract the target nucleic acid from the biological sample.

In another aspect, the methods of the invention further comprise performing a nucleic acid amplification following step (d) and detecting the amplified nucleic acid product. The nucleic acid amplification is carried out using PCR in some embodiments.

In some aspects, step (a) and step (b) are performed simultaneously.

In some aspects, the biological sample is whole blood, serum, plasma, sputum, saliva, urine, stool, cells, or microorganisms.

In some aspects, the transition metal salt is selected from the group consisting of a manganese salt or a zinc salt.

In some aspects, the alkaline earth metal salt is selected from the group consisting of a magnesium salt or a calcium salt.

In some aspects, the target nucleic acid is RNA. In some embodiments, the target nucleic acid is viral RNA.

In some aspects, the target nucleic acid is DNA. In some embodiments, the target nucleic acid is viral DNA.

In some aspects, the acidic buffer is citric acid buffer or acetic acid buffer.

In some aspects, the extraction solution comprises proteinase K, detergent, or a combination thereof.

In some aspects, the detergent is a cationic or non-ionic detergent. In some embodiments, the cationic detergent is cetrimonium bromide. In some embodiments, the non-ionic detergent is Triton X-100. In some embodiments, the non-ionic detergent is NP-40. In other embodiments, the detergent is a block copolymer (e.g., pluronic solution).

In some aspects, for example when the sample comprises Hepatitis B virus, the methods of the invention further comprise a step of heating the biological sample prior to separating the target nucleic acid from the lysate. In a typical embodiment, the biological sample is heated to a temperature between 50°C and 80°C, usually between 65°C and 75°C. The step of heating can be carried out for 30 seconds to 90 seconds or for 50 seconds to 70 seconds. In some embodiments, the heating step is carried out after the addition of the extraction solution and prior to the addition of the acidic buffer.

In some aspects, step (d) is conducted by adding magnetic particles to the lysate in step (c); separating the magnetic microparticles from the lysate using magnetic microparticle separation; adding a wash solution to the magnetic microparticles, and adding an elution buffer to the magnetic microparticles to elute the nucleic acid.

In one aspect, the invention provides a method for detecting an amplified nucleic acid product in a sample comprising a target nucleic acid and a nuclease, the method comprising:
(a) adding a transition metal salt, an alkaline earth metal salt, or combination thereof to the sample in an amount sufficient to inactivate the nuclease in the sample;
(b) adding an acidic buffer to the sample in an amount sufficient to reduce the pH of the sample to less than about pH 5;
(c) separating the target nucleic acid from the sample;
(d) performing a nucleic acid amplification reaction to produce the amplified nucleic acid product from the target nucleic acid;
(e) detecting the amplified nucleic acid product.

In one aspect, step (a) and step (b) are performed simultaneously.

In one aspect, the transition metal salt is selected from the group consisting of a manganese salt or a zinc salt. In one aspect, the alkaline earth metal salt is selected from the group consisting of a magnesium salt or a calcium salt.

In some aspects, the acidic buffer is citric acid buffer or acetic acid buffer.

In some aspects, the nucleic acid amplification reaction is a PCR process.

In some aspects, the target nucleic acid is RNA. In some embodiments, the target nucleic acid is viral RNA.

In some aspects, the target nucleic acid is DNA. In some embodiments, the target nucleic acid is viral DNA.

In one aspect, the invention provides the use of a kit for extracting nucleic acid from a biological sample comprising nucleic acids, nucleases and inhibitors comprising:
(a) an extraction solution;
(b) a transition metal salt solution, an alkaline earth metal salt solution, or combination thereof;
(c) an acidic buffer, and
(d) a wash solution
in a method according to the invention.

In some aspects, the extraction solution comprises proteinase K, detergent, or a combination thereof.

In some aspects, the transition metal salt is selected from the group consisting of a manganese salt or a zinc salt.

In some aspects, the alkaline earth metal salt is selected from the group consisting of a magnesium salt or a calcium salt.

In some aspects, the acidic buffer is citric acid buffer or acetic acid buffer.

In some aspects, the wash solution is trifluoroacetic acid solution or hydrochloric acid solution.

Other objects, features, and advantages of the present invention will be apparent to one of skill in the art from the following detailed description and figures.

### BRIEF DESCRIPTION OF THE DRAWINGS

**Figure 1** illustrates an embodiment of the invention described herein. It shows the steps of RNA extraction from a sample using divalent salt. The prelysis steps include steps 1-2. The lysis steps include steps 3-6. Removal of divalent salt, such as Mn2⁺ and other inhibitor of RNA extraction and PCR amplification occurs from step 5 with the addition of acidic buffer to step 9 after the final wash of the magnetic beads for nucleic acid capture. The RNA elution step includes step 10. Prior to performing PCR amplification (e.g., RT-PCR) the eluted RNA is treated with an acid such as HCl.
**Figure 2** shows the effect of low equimolar concentrations of divalent ions on viral RNA extraction of a sample spiked with HIV-1. Treatment with 40 mM MnCl₂ during RNA extraction yielded more HIV-1 RNA than treatments with 40mM MgCl₂ or CaCl₂.
**Figure 3** shows the effect of low equimolar concentrations of multivalent ions on viral RNA extraction of a sample spiked with HIV-1. Samples treated with 40mM MnCl₂ generated more HIV-1 RNA compared to those treated with either CaCl₂, MgCl₂, NH₄SO₄, or NH₄Cl₂. Adding 257.8 mM CaCl₂ to the sample was also effective for HIV-1 RNA isolation.
**Figure 4** shows the effect of high equimolar concentrations of multivalent ions on viral RNA extraction of a sample spiked with HIV-1. Samples were treated with 1.48M of CaCl₂, NaCl, MgCl₂, MnCl₂, ZnCl₂, NH₄Cl, NH₄(SO₄)₂, 229.6 M ZnCl₂ or no ion. The data revealed that at high equimolar concentrations, alkaline earth metal salts and transition metal salts perform similarly.
**Figure 5** shows a comparison of different divalent salts on viral RNA extraction of plasma samples spiked with HCV. The quantitative PCR data shows that extractions using Mn(OAc)₂ out-performed those with Mg(OAc)₂, Zn(OAc)₂ or MnCl₂. The results also suggest that extraction methods with 92.7 mM Zn(OAc)₂ can be effective.
**Figure 6** illustrates that the addition of divalent ions to a sample at the start of RNA extraction can preserve RNA integrity during prelysis steps (before the addition of Mn(OAc)₂ to plasma and after Mn(OAc)₂ addition) and lysis steps (addition of detergent such as Triton X-100, protease such as proteinase K, or magnetic bead binding buffer such as citric acid).
**Figure 7** illustrates that transition metal salts can be used in viral DNA extraction of a plasma sample spiked with EBV virus. Quantitative analysis of EBV DNA extraction methods using MgCl₂ or MnCl₂ is presented.
**Figure 8** shows quantitative comparison of DNA in samples treated with specific divalent salts. The levels of DNA were compared using Cp values determined by quantitative PCR.
**Figure 9** shows real-time quantitative PCR results comparing different extraction methods that included the addition of various concentrations of either CaCl₂ or MnCl₂ to clinical plasma samples spiked with HIV-1.

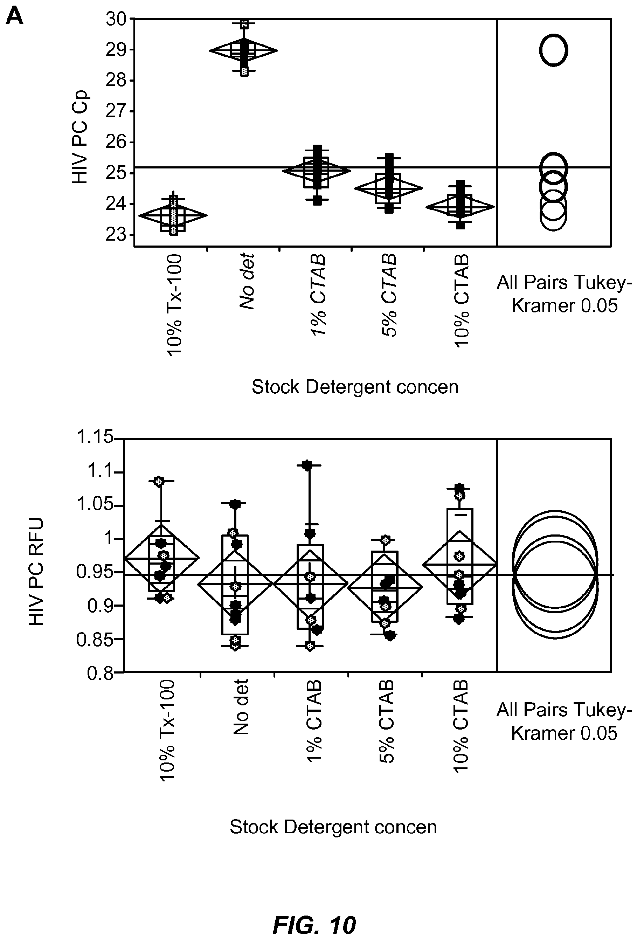

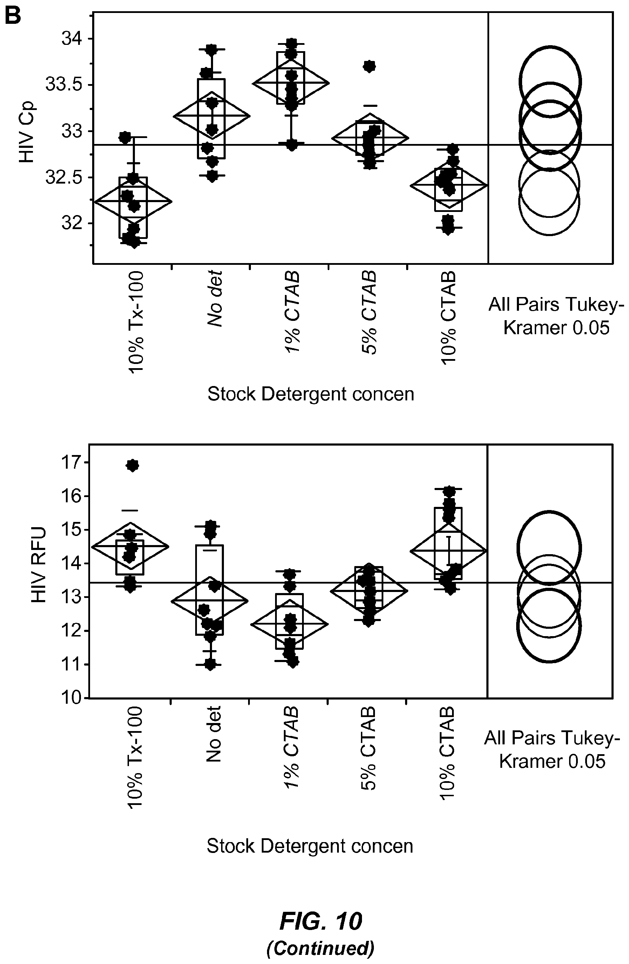

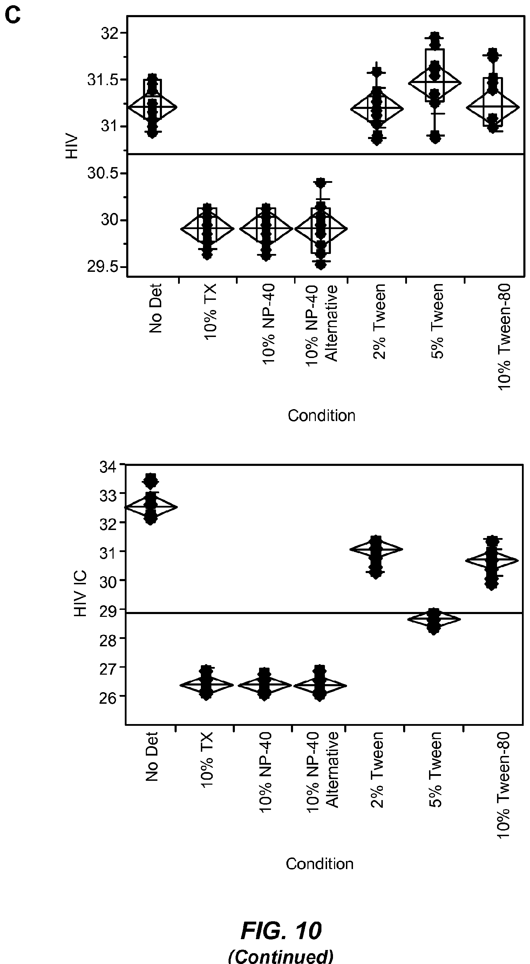

**Figures 10A****,** **10B****, and** **10C** show a comparison of various extraction methods using different detergents for the isolation of viral RNA from samples spiked with HIV-1. Figure 10A shows real-time quantitative PCR data for the HIV-1 process control. Viral RNA extraction with 10% Triton X-100 or 10% CTAB was comparable. Figure 10B shows that 10% Triton X-100 and 10% CTAB were most effective as measured by Cp value (Ct) for HIV-1 RNA extraction. Figure 10C shows that the addition of 10% Triton X-100, 10% NP-40 or 10% NP-40 alternative were equally effective for the isolation of HIV-RNA from a sample spiked with HIV-1.
**Figure 11** shows that extraction of HBV DNA from plasma samples is improved by incubating the lysis mixture containing CaCl₂ in a 70 °C incubator for 50 to 70 seconds.
**Figures 12A and 12B** show that extraction of 70 IU HBV DNA (Figure 12A) and 700,000 IU HBV DNA (Figure 12B) from serum samples is improved by incubating the lysis mixture containing CaCl₂ in a 70 °C incubator for 60 seconds.

### DETAILED DESCRIPTION OF THE INVENTION

### I. Introduction

The present invention is based, at least in part, on the discovery that the use of transition metal salts, alkaline metal salts, or combinations thereof (referred to here as "divalent salts"), along with low pH, during extraction of nucleic acids inactivates nucleases and inhibitors of amplification that may be present in biological samples. In addition, the methods of the invention can include the use of proteases (*e.g*., for samples that contain cells) to further inactivate the nucleases in the sample. The invention provides methods and compositions for effectively inactivating nucleases during extraction and/or amplification of nucleic acids from a biological sample. The invention also relates to a method of detecting an amplified nucleic acid product by inactivating nucleases in a biological sample.

The methods of the invention are typically used with samples comprising cells. In such methods, an extraction solution is used to lyse the cells and release nucleic acids from the cells. In that process, nucleases and inhibitors of amplification may be released at the same time. Without wishing to be bound by theory, it has been observed that transition metal salts and/or alkaline metal salts, especially at saturating concentrations, transiently inactivate the released nucleases by binding to specific charged amino acid residues on the protein. Subsequently, lowering the pH of the sample (for example to a pH below 5) results in loss of affinity of the divalent ions to the nucleases and the ions thus fall back into solution. However, at low pH, the nucleases are structurally deformed and get washed away during subsequent steps. As demonstrated herein, use of nuclease inactivation by divalent ions and protein denaturation at acidic pH (and optionally including addition of a protease) improves the extraction of nucleic acids from a variety of biological samples.

### II. Biological Samples

A biological sample useful in the present invention is any sample that contains, or is suspected of containing, target nucleic acids along with nucleases and/or inhibitors of amplification. In the typical embodiment, the biological sample comprises cells or microorganisms. Microorganisms include archaea, bacteria, fungi, Protista and viruses. The sample may be derived from a variety of biological sources. Such sources include whole tissues, including biopsy materials and aspirate, stool, cellular explants; whole blood, red blood cells, white blood cells, and body fluids such as lymph, urine, sputum, semen, secretions, eye washes and aspirates, lung washes and aspirates from a subject. The sample may also be *in vitro* cultured cells, including primary and secondary cells, and transformed cell lines, and cell culture media. A biological sample may also be derived from plant tissues, such as leaves, roots, or stems. In some embodiments, microorganisms (*e.g*., bacteria, fungi, algae, protozoa and viruses) may be present in the biological sample. In some embodiments, the biological sample is cell-free and contain virus. The terms "biological sample" and "sample" are used interchangeably.

The subject from which the biological sample is derived can be a human, a commercially significant mammal, including, for example, a monkey, cow, or horse. Samples can also be obtained from household pets, including, for example, a dog, cat or bird. In some embodiments, the subject is a laboratory animal used as an animal model of disease, for example, a mouse, a rat, a rabbit, a guinea pig, or a monkey.

In some embodiments, the target nucleic acid is RNA such as human mRNA, viral RNA, bacterial RNA, fungal RNA, algal RNA or protozoan RNA. In a typical embodiment, the target RNA is viral RNA.

In some embodiments, the target nucleic acid is DNA such as human DNA, viral DNA, bacterial DNA, fungal DNA, algal DNA or protozoan DNA. In a typical embodiment, the target DNA is viral DNA.

The nuclease which is inactivated in the methods of the invention can be any enzyme capable of cleaving the phosphodiester bonds between the nucleotide subunits of nucleic acids. Nucleases can be characterized as endonucleases, exonucleases, or both. Non-limiting examples of DNAse include DNase I, DNase II, DNase IV, restriction endonucleases, other endonucleases, and fragments thereof (that retain nuclease activity). Non-limiting examples of an RNase include a member of the RNase A family, RNase B, RNase C, RNase 1, RNase T1, RNase T2, RNase L, a member of the RNase H family, a member of the angiogenin RNase family, eosinophil RNase, a micrococcal nuclease, a member of the mammalian ribonuclease I family, a member of the ribonuclease 2 family, a messenger RNA ribonuclease, 5'-3' exoribonuclease, 3'-5' exoribonuclease, a decapping enzyme, a deadenylase, RNase P, RNase m, RNase E, RNase I,I* RNase HI, RNase HII, RNase M, RNase R, RNase IV, F; RNase P2,0, PIV, PC, RNase N, RNase II, PNPase, RNase D, RNase BN, RNase T, RNase PH, OligoRNase, RNase R, RNase Sa, RNase F1, RNase U2, RNase Ms, RNase St, and fragments thereof (that retain nuclease activity).

As noted above, the sample may also comprise inhibitors of amplification. Such inhibitors are typically inhibitors of PCR amplification. Inhibitors of amplification usually affect such assays through interaction with the target nucleic acid or interference with the DNA polymerase. Inhibitors can escape removal during the purification procedure by binding directly to nucleic acids in the sample. Alternatively, the inhibitors may inhibit PCR by reducing the availability of cofactors (for example, Mg²⁺) or otherwise interfering with their interaction with the DNA polymerase. Exemplary inhibitors include heme, hemoglobin, and lactoferrin and immunoglobins (in blood); bile salts (in feces); complex polysaccharides (in feces and plant material); melanin (in hair and skin); proteinases (in milk); myoglobin (in muscle tissue); humic acid (in soil and plant material); collagen (in tissues); and urea (in urine).

### III. Extraction of Nucleic Acids

As noted above, the present invention provides methods for extraction of target nucleic acids from a biological sample comprising nucleases and/or inhibitors of amplification. The divalent salts used in the methods of the invention can be salts of alkaline earth metals such as beryllium, magnesium, calcium, strontium, barium and radium. The divalent salts can also be salts of transition metals such as manganese, cobalt, nickel, copper, zinc, yttrium, sirconium, niobium, molybdenum, technetium, ruthenium, rhodium, palladium, silver, cadmium, lanthanum, hafnium, tantalum, tungsten, rhenium and osmium. The counter ion can be, for example, chloride, acetate, oxide, hydroxide, oxalate, carbonate, citrate, and trifluoroacetate.

Exemplary divalent salts useful in the present invention include magnesium chloride, calcium chloride, manganese acetate, manganese chloride, zinc acetate, and combinations thereof.

In a typical embodiment, hypermolar concentrations such as saturating amounts of divalent salts are conveniently used. Such an amount is sufficient to inactivate nucleases in the sample and thereby prevent nucleases from degrading the target nucleic acids. This amount will typically be a final concentration of at least about 250 mM to about 400 mM, and often up to about 500mM. In a typical embodiment, the amount of the divalent salt is at least about 250 mM.

The extraction methods may also include a step of adding an extraction solution to the biological sample in an amount sufficient to lyse cell, thereby forming a lysate. In some embodiments, the extraction solution comprises a protease, a detergent, or a combination thereof. The protease digests proteins (*e.g*., nucleases) released from the cells during lysis. Proteases useful for this purpose are well known to those of skill in the art. A protease can be any enzyme that catalyzes the cleavage of peptide bonds, e.g., in proteins, polypeptides, oligopeptides. Exemplary proteases include subtilases, subtilisins, and alkaline serine proteases. Subtilases are a family of serine proteases found in prokaryotic and eukaryotic organisms, such as, bacteria, fungi, and yeast. Subtilisins are bacterial subtilases that have broad substrate specificities. Subtilisins are relatively resistant to denaturation by chaotropes, such as urea and guanidine hydrochloride, and anionic surfactants, such as sodium dodecyl sulfate (SDS). Exemplary subtilisins include, but are not limited to: Proteinase K; Proteinase R; Proteinase T, Subtilisin A, Nagarse, Subtilisin B, and Thermitase. In a typical embodiment, Proteinase K is used in the methods of the invention. Those of skill will recognize that the use of proteases in nucleic acid extraction methods is well known and that the appropriate concentration of protease will depend upon a number of factors including the particular protease used and sample being analyzed. In a typical embodiment, the final protease concentration will be at least about 20 U/ml, usually up to about 40U/ml.

A detergent is typically used to disrupt viral, bacterial, cellular (*e.g*, derived from an animal, microbe, plant, human) membranes and release biological molecules such as protein, RNA and DNA from inside the cell or viral particle. The lysate thus formed is typically at or near neutral pH (*e.g*., about pH 7). The use of detergents in nucleic acid extraction methods is also well known. Those of skill will recognize that the final detergent concentration will depend upon a number of factors including the particular detergent used and sample being analyzed. In a typical embodiment, the final detergent concentration will be at least about 0.1%, usually up to about 5%.

Detergents useful for this purpose are well known to those of skill in the art. In some embodiments, the detergent is a cationic surfactant. Cationic surfactants may contain quaternary amines or tertiary amines. Exemplary cationic surfactants include cetyltrimethylammonium bromide (CTAB), cetyltrimethylammonium chloride (CTACl), dodecyltrimethylammonium bromide (DTAB,), dodecyltrimethylammonium chloride (DTACl), octyl trimethyl ammonium bromide, tetradecyltrimethylammonium bromide (TTAB), tetradecyltrimethylammonium chloride (TTACl), dodecylethyldimethylammonium bromide (DEDTAB), decyltrimethylammonium bromide (D10TAB), dodecyltriphenylphosphonium bromide (DTPB), octadecylyl trimethyl ammonium bromide, stearoalkonium chloride, olealkonium chloride, cetrimonium chloride, alkyl trimethyl ammonium methosulfate, palmitamidopropyl trimethyl chloride, quaternium 84. Exemplary ternary amine surfactants include octyldimethylamine, decyldimethylamine, dodecyldimethylamine, tetradecyidimethylamine, hexadecyldimethylamine, octyldecyldimethylamine, octyldecylmethylamine, didecylmethylamine, dodecylmethylamine, triacetylammonium chloride, cetrimonium chloride, and alkyl dimethyl benzyl ammonium chloride. Additional classes of cationic surfactants include phosphonium, imidzoline, and ethylated amine groups. In a typical embodiment, the cationic detergent is CTAB.

In some embodiments, the detergent is a non-ionic detergent. Non-limiting examples of a non-ionic detergent include NP-40, Triton X-100, Triton X-114, Tween-20, Tween-80, Brij-35, Brij-58, octyl glucoside, octyl thioglucoside, block copolymer (e.g., pluronic solution). In a typical embodiment, the non-ionic detergent is Triton X-100. In other embodiments, the non-ionic detergent is NP-40.

In some embodiments, the detergent is a zwitterionic detergent. Non-limiting examples of a Zwitterionic detergent include 3-[(3-Cholamidopropyl)dimethylammonio]-1-propanesulfonate (CHAPS), 3-[(3-Cholamidopropyl)dimethylammonio]-2-hydroxy-1-propanesulfonate (CHAPSO), N-Decyl-N,N-dimethyl-3-ammonio-1-propanesulfonate (Sulfobentaine 3-10), N-Tetradecyl-N,N-dimethyl-3-ammonio-1-propanesulfonate (Sulfobetaine 3-14), N-Dodecyl-N,N-dimethyl-3-ammonio-1-propanesulfonate (Sulfobetaine 3-12), Amidosulfobetaine-14, Amidosulfobetaine-16, 4-n-Octylbenzoylamido-propyl-dimethylammonio sulfobetaine, and 3-(1-Pyridino)-1-propane sulfonate (Non-Detergent Sulfobetaine 201).

In some embodiments, the detergent is formulated with an antifoam (defoamer) agent. The antifoam is a chemical additive that reduces and/or hinders the formation of foam in a liquid. Non-limiting examples of an antifoam agent include organic antifoam (e.g., antifoam 204, antifoam O-30) and silicone-based antifoam (e.g., antifoam A, antifoam B, antifoam C, antifoam Y-30). In a typical embodiment, the final concentration of an antifoam agent will be at least about 0.001% usually up to about 0.5%.

In some embodiments, the divalent salt and the extraction solution are added to the biological sample simultaneously. In some embodiments, the divalent salt is added to the sample prior to the addition of the extraction solution or any component of the extraction solution during the prelysis phase of the extraction.

In the methods of the invention, an acidic buffer is added to the lysate to an amount sufficient to reduce the pH to less than pH 5, typically less than about 4, usually less than about pH 3, and often as low as about pH 1. Under low pH conditions, the residual nucleases are further denatured, remain inactive and are unable to degrade nucleic acids. Also, the transition metal ions and alkaline earth metal ions lose affinity for nucleases and other metalloproteins in the lysate, and fall into solution. Thus low pH conditions facilitate the removal of the metal salts and other inhibitors during subsequent wash steps. Those of skill will recognize that the final acidic buffer concentration will depend upon a number of factors including the particular buffer used and sample being analyzed. In a typical embodiment, the final acidic buffer concentration will be at least about 10mM usually up to about 250mM.

In some embodiments, the acidic buffer is an acetic acid buffer or citric acid buffer. Other examples of acidic buffers useful in the invention include oxalic acid, hydrofluoric acid, trifluoroacetic acid, glycine, pyruvic acid, lactic acid, and any acidic amino acids at a concentration of at least 10 mM, usually up to about 250 mM.

The experimental results provided here show that, in the case of samples comprising Hepatitis B virus, target nucleic acid extraction can be further improved by heating the biological sample during the methods of the invention. Thus, in some embodiment, the methods of the invention further comprise a step of heating the biological sample prior to separating the target nucleic acid from the lysate. In a typical embodiment, the biological sample is heated to a temperature between 50°C and 80°C, usually between 65°C and 75°C. The step of heating can be carried out for 30 seconds to 90 seconds or for 50 seconds to 70 seconds. In some embodiments, the heating step is carried out after the addition of the extraction solution and prior to the addition of the acidic buffer.

Thus, in one aspect, the present invention provides a method of inactivating nuclease during extraction of target nucleic acids in a biological sample comprising cells. The method involves the following steps: (1) inactivating nuclease by the addition of transition metal salts, alkaline earth metal salts, or combinations thereof; (2) lysing cells of the biological sample using detergents (e.g., cationic or non-ionic detergents), proteinase K, or combinations thereof to form a lysate; (3) reducing the pH of the lysate to a pH of about 1 to about 5 using an acidic buffer; (4) capturing target nucleic acids at low pH and removing metal salts; and (5) removing impurities (*e.g*, nucleases and inhibitors of PCR amplification or other downstream process) at low pH; and (6) releasing the target nucleic acids under alkaline conditions. In some embodiments, at least two of the steps can occur simultaneously. In some embodiments, two or more (e.g., 3, 4, or 5) steps can occur simultaneously. In some embodiments, the method comprises a plurality of steps that can occur simultaneously, followed by another plurality of steps that can occur simultaneously.

After the addition of the acidic buffer, the target nucleic acid is separated from the lysate using standard techniques well known to those of skill in the art. In some embodiments, magnetic beads that bind the target nucleic acid (either specifically or non-specifically) are used (*see e.g*., Rudi et al. BioTechniques 22(3) 506-511, 1997).

For example, in some embodiments, the methods of the invention include a step of adding 0.25 to 2.5 mg magnetic microparticles to the lysate, separating the magnetic particles from the lysate using magnetic microparticle separation; adding a washing solution to the magnetic particles; and adding an elution buffer to the magnetic microparticles to elute the nucleic acid. In some embodiments, the step is performed for example, using solid phase reversible immobilization paramagnetic bead-based technology.

### IV. Detection of the target nucleic acids

Methods of detecting and analyzing target nucleic acids are well known to those of skill in the art and are not described in detail here. In a typical embodiment, the target nucleic acid is amplified prior to further analysis. Amplification can be carried out either before or after the target nucleic acid is separated from the lysate.

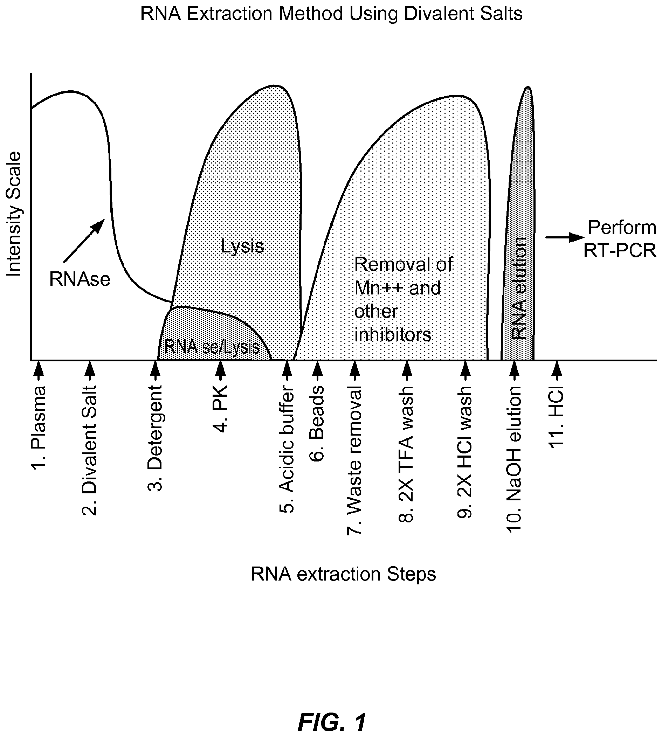

An exemplary embodiment of a method of the invention is illustrated in Figure 1 and includes the following steps: (1) obtaining a sample; (2) adding MgCl₂, CaCl₂, Mn(OAc)₂, MnCl₂, Zn(OAc)₂, or combinations thereof to a biological sample to a final concentration of up to 500mM; (3 and 4) adding Triton X-100 or CTAB and proteinase K to a final concentration of up to 10% and 40 U/ml, respectively; (5) reducing the pH of the resulting lysate to less than about pH 5 by adding either citric acid buffer or acetic acid buffer to a final concentration of up to 160 mM; (6) capturing the target nucleic acids on a solid support by adding 2 mg of magnetic microparticles; (7) separating the magnetic microparticles using a magnet and removing waste according to manufacturer's instructions; (8) separating the beads from the magnet and washing the magnetic microparticles with a wash solution such as 900 µl of 1-100mM trifluoroacetic acid solution and/or 1-100 mM HCl, pH 1-5 at least once; (9) washing the beads with 1-100 mM HCl, pH 1-5 at least once; (10) eluting the target nucleic acids from the magnetic microparticles with an elution solution such as 25 µl of 75 mM NaOH and 50 mM Tris, pH 8.0; (11) neutralizing the eluate comprising target nucleic acids with 4 µl of 150mM HCl; and (12) performing quantitative nucleic acid amplification such as real-time quantitative PCR using the eluate.

Without being bound to any particular theory, it is believed that the addition of divalent salts in step 2 causes transient inactivation of nucleases present in the sample. Due to the low pH in the sample at step 5, divalent salts lose affinity for nucleases and other metalloproteins. This step and subsequent steps 7-9 facilitate the removal of divalent salts and other inhibitors of nucleic acid amplification and other downstream process.

Thus, in some embodiments, the method further comprises performing a nucleic acid amplification before or after the step of isolating the target nucleic acid. Nucleic acid amplication includes polymerase chain reaction (PCR) and variants thereof (e.g., allele-specific PCR, assembly PCR, asymmetric PCR, methylation-specific PCR, multiplex-PCR, nested PCR, quantitative PCR, reverse transcription PCR, and real-time quantitative PCR. In some embodiments of the present invention, nucleic acid amplification is PCR.

In some embodiments, the method comprises detecting an amplified nucleic acid product generated from the nucleic acid amplification. In some embodiments, a signal generated during the amplification process is monitored, wherein the signal is related to the amount of amplified nucleic acid and/or target nucleic acid in the reaction. The signal can be fluorescence or another modality that is detectable and quantifiable.

In an exemplary embodiment, the steps of nucleic acid amplification and detection of the resulting amplified nucleic acid product are performed in a PCR system. Such a system takes a prepared and sealed reaction vessel and performs a complete real time polymerase chain reaction analysis, thermal cycling the sample multiple times and reporting the intensity of emitted fluorescent light at each cycle.

An automated instrument for the determination of nucleic acids according to an embodiment of the present invention is described in detail in WO 2012/012779. Thus, embodiments of the invention include a fully automated, random access system for determining specific target nucleic acid sequences in a biological sample. The system includes consumables incorporating necessary reagents for performing a variety of assays, reaction sites, and transfer devices. Sufficient storage space for consumables is provided on the system to permit it to run with minimal operator intervention for an extended time.

The system can combine two functions: sample preparation in the form of isolation of nucleic acids from the biological sample, and detection of specific target nucleic acid sequences within these isolated nucleic acids. Towards this end, the system can have at least two distinct functional areas: one including instrumentation to process samples using the consumables and a second including the instrumentation and reagents for nucleic acid amplification and detection. These are integrated in a single unit to provide a system that performs major functions of sample handling, nucleic acid isolation, and amplification and detection, plus supporting functions of supply and consumable management, information management, and maintenance.

Combining these functions into a single, highly automated, self-contained system provides seamless integration of molecular diagnostics into the workflow of the clinical laboratory. Such a system allows one of skill to perform all steps of nucleic acid determination to produce clinically acceptable results without the need for user intervention. The system advantageously allows users to load samples as they become available, and to perform determinations on those samples as dictated by the needs of the patient and their physician, without constraints on sample or analyte order being imposed by the system.

### V. Kits

The reagents useful in the methods of the invention can also be produced in the form of kits. Accordingly, all discussion of the reagents used in the methods of the invention applies *mutatis mutandis* to define the kits used in the methods of the invention. Such kits are a packaged combination comprising, for example, the basic elements of for extracting nucleic acid from a biological sample comprising nucleic acids, nucleases and inhibitors. The kits can comprise:
(a) an extraction solution;
(b) a transition metal salt solution, an alkaline earth metal salt solution, or combination thereof;
(c) an acidic buffer, and
(d) a wash solution.

In some embodiments, the extraction solution comprises proteinase K, detergent, or a combination thereof. In some embodiments, the detergent is a cationic or non-ionic detergent. In some embodiments, the cationic detergent is cetrimonium bromide. In some embodiments, the non-ionic detergent is Triton X-100.

In some aspects, the transition metal salt is selected from the group consisting of a manganese salt or a zinc salt.

In some aspects, the alkaline earth metal salt is selected from the group consisting of a magnesium salt or a calcium salt.

In some aspects, the acidic buffer is citric acid buffer or acetic acid buffer.

In some aspects, the wash solution is trifluoroacetic acid solution, hydrochloric acid solution, oxalic acid solution, pyruvic acid solution, lactic acid solution, or a solution comprising an acidic amino acid..

### EXAMPLES

### Example 1: RNA extraction method using divalent ions and multivalent ions

This example illustrates the methods described herein for extracting RNA from a sample using divalent ions, protease, detergent and low pH. The performance of nucleic acid extraction methods comprising multivalent salts at either low or high equimolar concentrations was evaluated by real-time quantitative PCR. In these studies, mean HIV-1 Ct, mean RFU and/or process control Ct were evaluated for each extraction method tested. A lower mean HIV-1 Ct value and/or a lower process control Ct value correlate to the presence of more extracted HIV-1 RNA.

The method of extracting RNA from a plasma sample is described herein and presented in Figure 1. Briefly, K2-plasma samples containing 4,000 copies of HIV-1 per ml and a 1:10,000 dilution of a HIV-1 process control (e.g., Sindbis HIV RNA control) were treated with one of the divalent salt conditions being tested. A process control serves as a positive control of nucleic acid extraction and PCR amplification. Next, cells and microorganisms (HIV-1 virus) of the sample were lysed using Triton X-100 and proteinase K to form a lysate. Afterwards the pH of the lysate was reduced to an acidic pH using an acidic buffer. Nucleic acids were captured using magnetic beads and a binding buffer such as citric acid. Inhibitors of PCR amplification or other downstream process, as well as divalent ion salt were removed through a series of washing with washing solutions containing trifluoroacetic acid or hydrochloric acid. Captured nucleic acids were eluted from the magnetic beads using an alkaline buffer such as sodium hydroxide. The eluted RNA was treated with HCl prior to PCR amplification. The performance of the extraction method was evaluated by real time quantitative PCR (RT-PCR) to quantitate the presence of HIV-1 RNA. In Figure 1 the steps of RNA extraction are depicted on the x-axis of the graph, and the activity of particular processes of the extraction (e.g., RNase activity, cell lysis, removal of Mn²⁺ and other inhibitors, and RNA elution) are plotted along the y-axis.

Nine extraction methods using low equimolar concentrations of different multivalent salts were compared. The following low equimolar conditions were tested: a) 40 mM of CaCl₂; b) 40 mM MgCl₂; c) 40mM MnCl₂; d) 40mM ammonium chloride (NH₄Cl₂); e) 40mM ammonium sulfate (NH₄SO₄); f) 40 mM CaCl₂ and 40 mM NH₄Cl; g) 257.8 mM CaCl₂ and 40 mM NH₄Cl; h) 257.8 mM CaCl₂ and 175 mM NH₄Cl; and i) 257.8 mM of CaCl₂. It should be noted that the molar concentration of the multivalent ion salt refers to the final concentration at the level of binding.

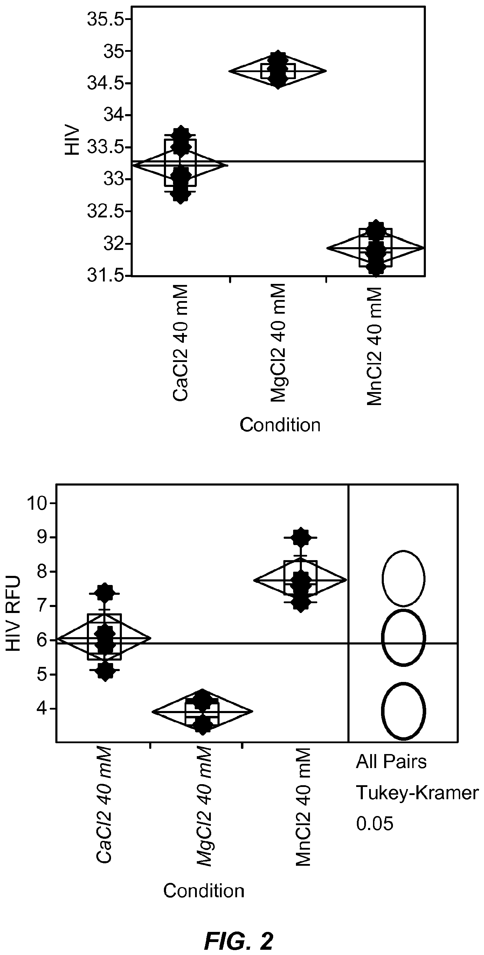

Figure 2 illustrates the results obtained from the comparison of the divalent salt conditions, e.g., 40 mM of CaCl₂, MgCl₂ and MnCl₂. At a low divalent ion concentration, MnCl₂ performed better than CaCl₂ and MgCl₂, as represented by the lower mean HIV Ct value and highest mean RFU.

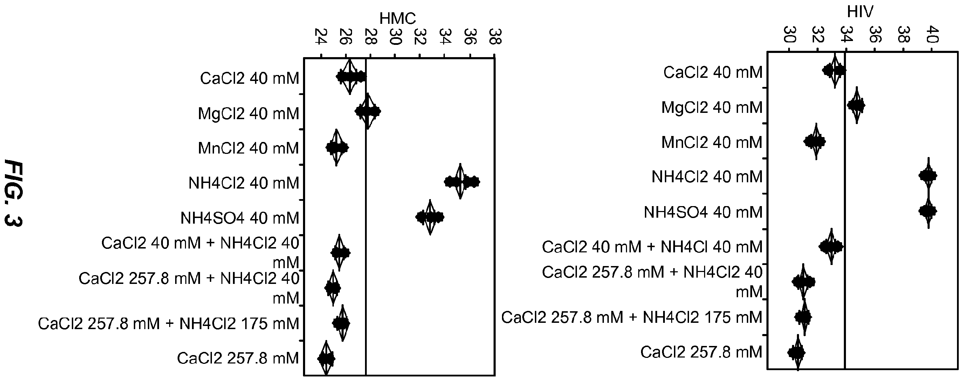

Figure 3 depicts the performance results of all 9 multivalent salt conditions tested. Performance analysis was based on mean HIV-1 Ct (HIV) and process control Ct (HIVIC) from each condition. The results show that the extraction method with MnCl₂ generated the lowest mean HIV-1 Ct and process control Ct. Thus, the extraction method using MnCl₂ performed better than the other methods tested.

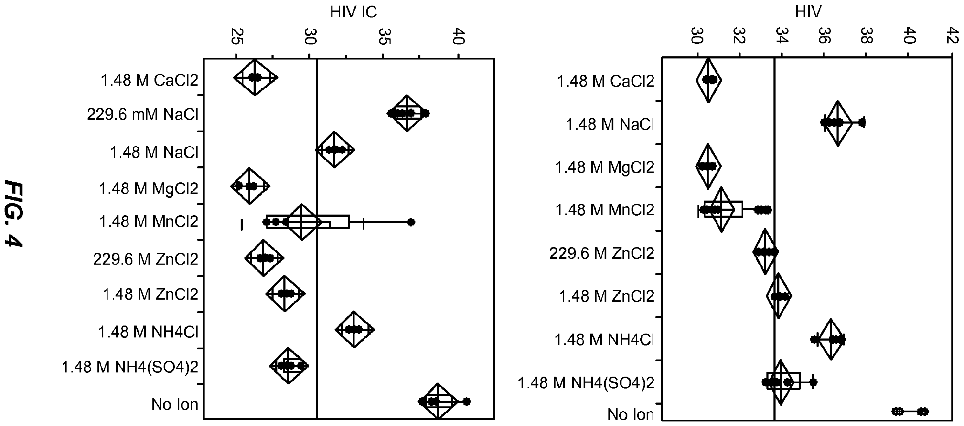

Next, nine extraction methods using high equimolar concentrations of different multivalent salts were compared. Similar to the testing of low equimolar concentrations of multivalent salts, 4,000 copies of HIV-1 per ml of K2-plasma along with standard concentration of a process control (PC) were extracted with 350 µl of various high equimolar concentrations of multivalent salts. The following high equimolar conditions were used: 1) 1.48 M calcium chloride (1.48M CaCl₂); 2) 1.48M sodium chloride (1.48 M NaCl); 3) 1.48 M magnesium chloride (1.48 M MgCl₂); 4) 1.48 M manganese chloride (1.48 M MnCl₂); 5) 229.6 mM zinc chloride (229.6 M ZnCl₂); 6) 1.48 M zinc chloride (1.48 M ZnCl₂); 7) 1.48 M ammonium chloride (1.48 M NH₄Cl); 8) 1.48 M ammonium sulfate (1.48 M NH₄(SO₄)₂; or 9) no specific ions (no ion). It should be noted that the molar concentration refers to the stock concentration used, and thus, the final concentration of the multivalent salt at binding was subject to a 5.74-fold dilution. The performance of nucleic acid extraction of plasma using different concentration of multivalent salts was evaluated based on lowest mean HIV-1 Ct (HIV) and process control Ct (HIVIC) from each condition. The results show that at high equimolar concentrations, alkaline earth metal salts and transition metal salts performed similarly (Figure 4).

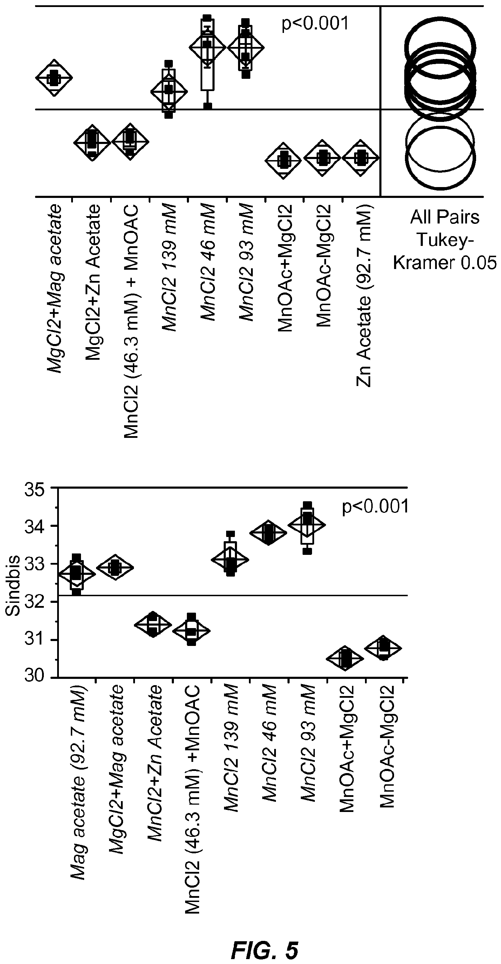

To determine if specific metal ions of the multivalent salt improved the extraction of target nucleic acids, methods containing either magnesium salts, manganese salts or zinc salts were compared. 2,000 copies of Hepatitis C virus (HCV) per ml of K2-EDTA plasma along with a standard concentration of PC (Sindbis) were extracted following the method depicted in Figure 1. At step 2, one of the following salt concentrations (final concentration) was added: a) 92.7 mM magnesium acetate; b) 46.3 mM magnesium chloride and 140 mM magnesium acetate c) 46.3 mM magnesium chloride and 140 mM zinc acetate; d) 46.3 mM manganese chloride and 140 mM manganese acetate; e) 46 mM MnCl₂;f) 93 mM MnCl₂; g) 139 mM MnCl₂; h) 140 mM manganese acetate and 46.3 mM magnesium chloride; i) 140 mM manganese acetate; and j) 92.7 mM Zinc acetate. The performance of the nucleic acid extraction was evaluated based on lowest mean HCV Ct (HCV) and PC Ct (Sindbis) from each condition. The results show that RNA extraction methods with a combination of either transition metal salts or alkaline earth metal salts with acetate ions performed the best (Figure 5).

### Example 2: Analysis of RNA Integrity During Lysis Steps

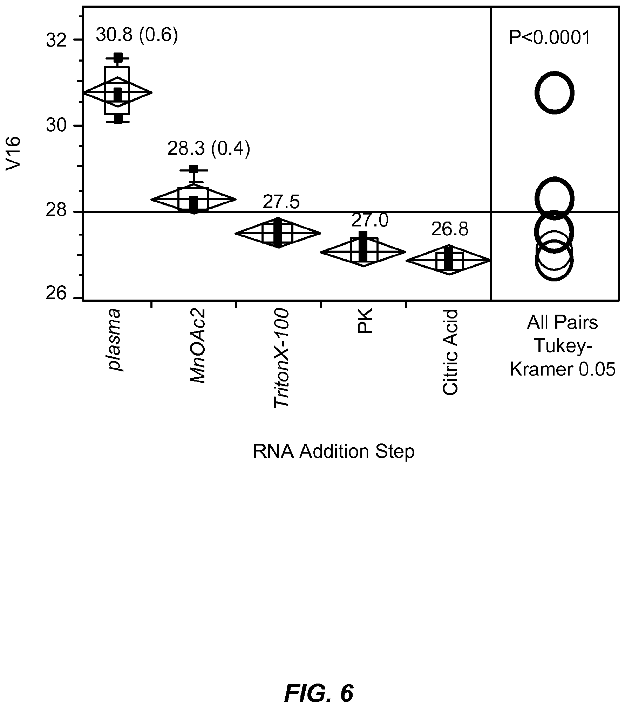

This example illustrates the effect on RNA integrity of HIV-1 genomic RNA during RNA extraction of K2 plasma samples treated with Mn(OAc)₂. In the study 20,000 copies of HIV genomic RNA were added at different steps of extraction. RNA integrity was estimated by real-time RT-PCR. The results show that the addition of Mn(OAc)₂ to a sample at the start of RNA extraction preserved RNA integrity during prelysis steps (before the addition of Mn(OAc)₂ to plasma and after Mn(OAc)₂ addition) and lysis steps (addition of detergent such as Triton X-100, protease such as proteinase K, or magnetic bead binding buffer such as citric acid). The addition of genomic RNA prior to Mn(OAc)₂ resulted in a 94% loss of RNA, as calculated from a 4 Ct delay (Figure 6). Yet, the addition after Mn(OAc)₂ led to a 82% protection of genomic RNA, as determined by a 2.5-3.5 Ct difference. RNA integrity was highest when the sample was spiked with RNA after the PK, as 90% of the RNA was preserved.

### Example 3: Use of divalent salts in DNA extraction

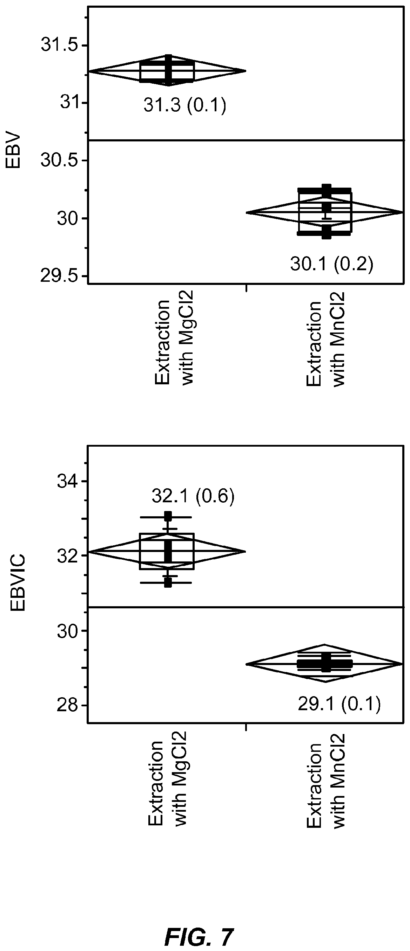

This example illustrates that transition metal salts are effective for viral DNA extraction of plasma samples spiked with Epstein-Barr virus (EBV). The effect of adding either manganese chloride or magnesium chloride (final concentration or 257 mM) during the initial step of DNA extraction to a plasma sample spiked with Epstein Barr virus (4,000 copies per ml) and a process control was evaluated by real-time quantitative PCR. The data shows that MnCl₂ was more effective than MgCl₂ for extracting viral DNA, as determined by Ct values for EBV and the process control (EBVIC). Figure 7 illustrates the quantitative analysis comparing EBV DNA extraction methods with MgCl₂ and MnCl₂.

### Example 4: Extraction of C. difficile DNA from a Biological Sample

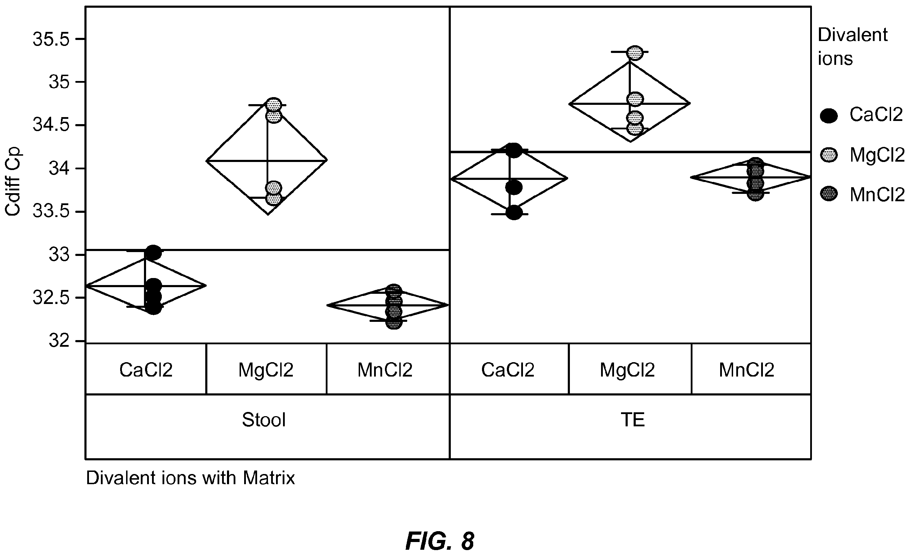

This example illustrates that divalent salts can be used in the extraction of bacterial DNA from samples containing bacteria. Samples comprised of 100 cells of *C. difficile* 027 (NCTC 13366) in 250 µl or synthetic stool swab (stool) or Tris EDTA buffer (TE). Prior to the addition of the extraction solution, 1.48 M of either CaCl₂, MgCl₂ or MnCl₂ were added to the samples. DNA was extracted from the samples using an automated nucleic acid extraction system. Target *C. difficile* DNA was measured using quantitative PCR amplification. The results show that samples treated with CaCl₂ and MnCl₂ have lower Ct values compared to samples treated with MgCl₂ (see, Figure 8). The data shows that the addition of CaCl₂ or MnCl₂ to a sample is effective for extracting bacterial DNA present in limiting quantities in the sample.

### Example 5: Titration of MnCl₂ and CaCl₂ for RNA Extraction

This example illustrates that an alkaline earth metal salt or a transition metal salt can be used to extract RNA from clinical samples. In this study we compared the effect of various concentrations (e.g., 64.6 mM, 128.9mM, 180mM and 257.8 mM final concentration) of CaCl₂ and MnCl₂ on HIV-1 RNA extraction of clinical samples spiked with 5,000 copies of HIV-1/ ml of plasma. The divalent salts were added to the clinical sample before the extraction solution.

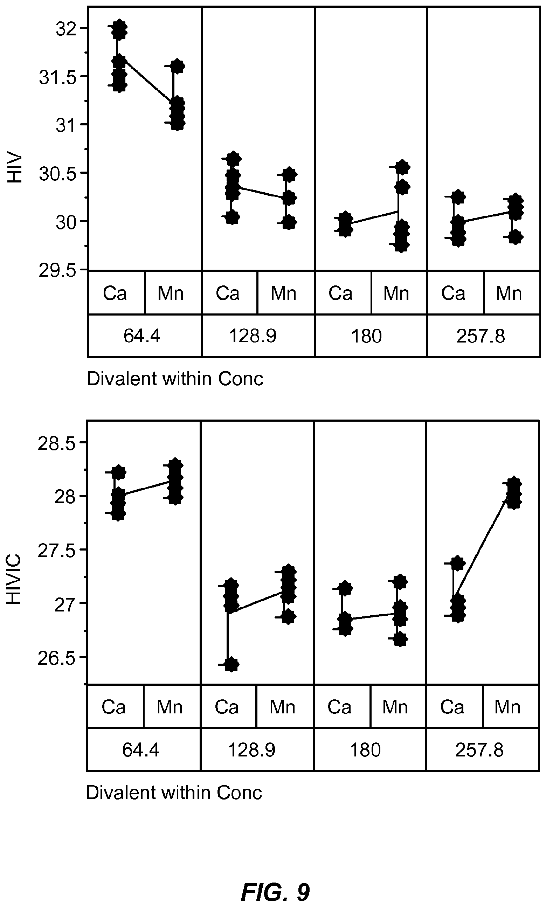

Real-time quantitative PCR was performed to measure the level of extracted HIV-1 RNA isolated from each condition. An HIV-1 process control comprising of HIV RNA was used as a positive control of RNA extraction and PCR amplification. The results shows that 64 mM MnCl₂ was more effective than 64mM CaCl₂ for RNA extraction (see, Figure 9). At higher concentrations (e.g., 128.9 mM and 180mM), the divalent salts performed similarly. Yet, at the highest salt concentration, the Ct value of the process control (HIVIC) was higher with MnCl₂ than CaCl₂.

### Example 6: Use of CTAB in HIV RNA Preparation

This example illustrates the effect of various detergents on viral RNA extraction of samples containing HIV-1. In the first assay ionic and cationic detergents were compared. Samples were spiked with 2,000 copies of HIV-1/ ml along with an HIV-1 RNA process control (e.g., Sindbis/HIV RNA). RNA was extracted with 200 µl of either 10% Triton X-100, 1% cetyltrimethylammonium bromide (CTAB), 5% CTAB, 10% CTAB, or no detergent in a total sample volume of 2,010 µl. The performance of the different extraction conditions were evaluated based on real-time quantitative PCR analysis. In particular, low mean HIV-1 and HIV process control Ct values (HIV Cp and HIV PC Cp, respectively), and high mean amplitude of HIV-1 and HIV-1 process control (HIV RFU and HIV PCR RFU, respectively) indicate effective viral RNA extraction. The results show that extraction conditions with 10% Triton X-100 or 10% CTAB performed similarly and were most effective compared to the other conditions tested (see, Figures 10A and 10B).

In the second assay the performance of various non-ionic detergents was analyzed. HIV-1 RNA was extracted from samples spiked with 10,000 copies of HIV/ ml of sample and a HIV-1 process control at 1:10,000 dilution. The non-ionic detergents included 10% Triton X-100, 10% NP-40, 10% NP-40 alternative, 2% Tween, 5% Tween, 10% Tween-80, and no detergent. The data shows that viral RNA extraction with 10% Triton X-100, 10% NP-40 and 10% NP-40 alternative were comparable (see, Figure 10C). These extraction methods isolated similar levels of HIV-RNA, as determined by Ct value.

### Example 7: Extraction of Hepatitis B DNA from a Biological Sample

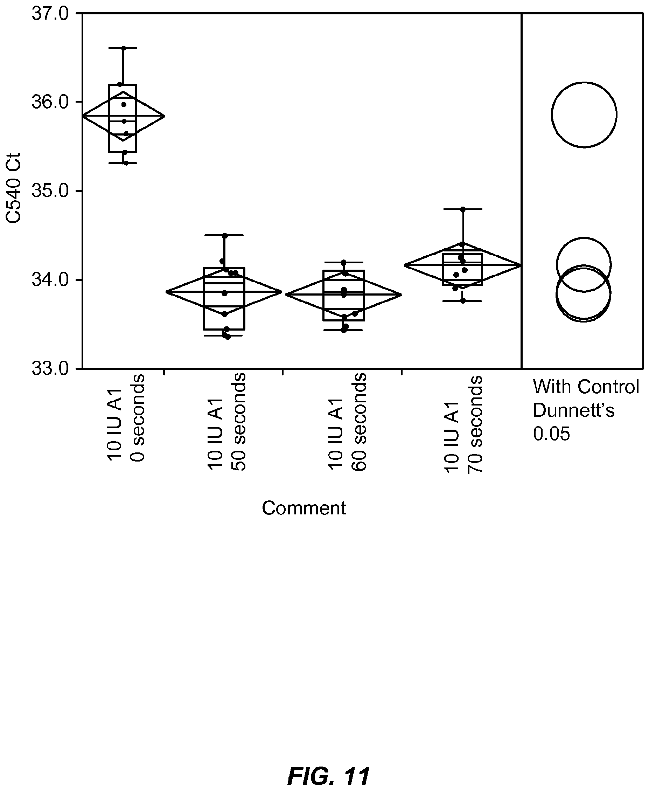

This example illustrates that CaCl₂ can be used in the extraction of Hepatitis B viral DNA from samples containing such viruses and that DNA yield from extractions using CaCl₂ can be increased when used in combination with heat. Samples comprised of 7 International Units Hepatitis (IU) B virus in 700 µl K2EDTA plasma (Figure 11). Lysis of Hepatitis B virus was achieved by the addition of 1.48 M CaCl₂, 10% Triton-X 100 and 800 Units/µl protease. The mixture was incubated for 0 seconds, 50 seconds, 60 seconds or 70 seconds in a heater set at 70 °C and then 4.7 M acetate buffer was added followed by 2 mg/ml magnetic particles. The DNA bound to the particles was purified by three washes and then dissociated from the particles by the addition of heated 75 mM NaOH. The aforementioned extraction and purification of Hepatitis B DNA was performed on an automated nucleic acid extraction system. Target Hepatitis B DNA was measured using quantitative PCR amplification. The results show that samples treated with CaCl₂ and heat are associated with lower Ct values compared to samples extracted in the absence of heat (see, Figure 11). The data show that the use of CaCl₂ is effective for extracting viral DNA present in limiting quantities in a biological sample. In addition, the data show that the use of heat in combination with CaCl₂ increases the effectiveness of the extraction resulting in greater DNA yields.

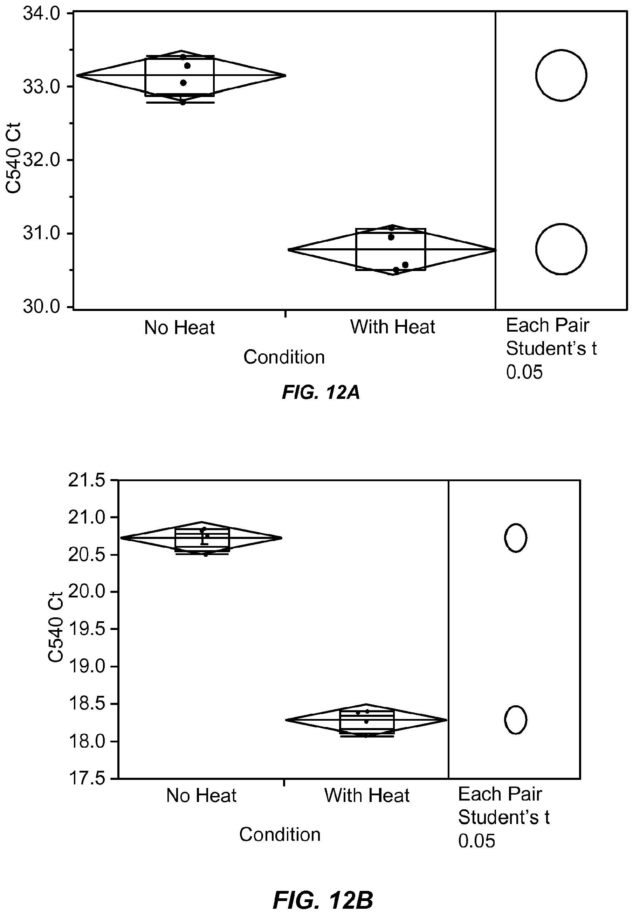

The chemical process described above can also be used to extract Hepatitis B DNA from serum. Samples comprised of 70 or 700,000 International Units Hepatitis B virus in 700 µl serum, Figure 12A and Figure 12B, respectively. Lysis of Hepatitis B virus was achieved by the same chemical process described above using a 60 second incubation time in the 70 °C heater. The results show that samples treated with CaCl₂ and heat are associated with lower Ct values compared to samples extracted in the absence of heat (see, Figure 12). The data show that the use of CaCl₂ is effective for extracting viral DNA present in limiting or abundant quantities in a serum sample. In addition, the data shows that the use of heat in combination with CaCl₂ increases the effectiveness of the extraction resulting in greater DNA yields.

## Claims

1. A method for extraction of a target nucleic acid from a biological sample comprising cells or microorganisms, the method comprising:
(a) adding a transition metal salt, an alkaline earth metal salt, or combination thereof to the biological sample in an amount sufficient to inactivate nucleases in the sample;
(b) adding an extraction solution to the biological sample in an amount sufficient to lyse the cells or microorganisms, thereby forming a lysate;
(c) adding an acidic buffer to the lysate to an amount sufficient to reduce the pH of the lysate to between pH 1 to pH 5; and
(d) separating the target nucleic acid from the lysate to extract the target nucleic acid from the biological sample.

2. The method of claim 1, further comprising performing a nucleic acid amplification, optionally using PCR, following step (d) and detecting the amplified nucleic acid product.

3. The method of any one of claims 1 to 2, wherein the biological sample is whole blood, serum, plasma, sputum, saliva, urine, stool, or tissue.

4. The method of any one of claims 1 to 3, wherein the target nucleic acid is DNA, optionally viral DNA.

5. The method any one of claims 1 to 4, wherein the extraction solution comprises proteinase K, detergent, or a combination thereof.

6. The method of claim 5, wherein the detergent is cationic detergent, optionally cetrimonium bromide.

7. The method of claim 5, wherein the detergent is non-ionic detergent, optionally Triton X-100.

8. The method of any one of claims 1 to 7, wherein step (d) is conducted by adding magnetic particles to the lysate in step (c); separating the magnetic microparticles from the lysate using magnetic microparticle separation; adding a wash solution to the magnetic microparticles, and adding an elution buffer to the magnetic microparticles to elute the nucleic acid.

9. A method for detecting an amplified nucleic acid product in a sample comprising a target nucleic acid and nucleases, the method comprising:
(a) adding a transition metal salt, an alkaline earth metal salt, or combination thereof to the sample in an amount sufficient to inactivate the nucleases in the sample;
(b) adding an acidic buffer to the sample in an amount sufficient to reduce the pH of the sample to less than pH 5;
(c) separating the target nucleic acid from the sample;
(d) performing a nucleic acid amplification reaction to produce the amplified nucleic acid product from the target nucleic acid;
(e) detecting the amplified nucleic acid product.

10. The method of claim 9, wherein the nucleic acid amplification reaction is a PCR process and/or wherein the target nucleic acid is DNA, optionally viral DNA.

11. The method of any one of claims 1-10, wherein:
(a) step (a) and step (b) are performed simultaneously; and/or
(b) the transition metal salt is selected from the group consisting of a manganese salt or a zinc salt; and/or
(c) the alkaline earth metal salt is selected from the group consisting of a magnesium salt or a calcium salt; and/or
(d) the acidic buffer is citric acid buffer or acetic acid buffer; and/or
(e) the target nucleic acid is RNA optionally viral RNA.

12. Use of a kit for extracting a nucleic acid from a biological sample comprising nucleic acids, nucleases and inhibitors comprising:
(a) an extraction solution;
(b) a transition metal salt solution, an alkaline earth metal salt solution, or combination thereof;
(c) an acidic buffer, and
(d) a wash solution;
in a method according to claim 1.

13. The use of claim 12, wherein
(a) the extraction solution comprises proteinase K, a detergent, or a combination thereof and/or
(b) the transition metal salt is selected from the group consisting of a manganese salt or a zinc salt and/or
(c) the alkaline earth metal salt is selected from the group consisting of a magnesium salt or a calcium salt; and/or
(d) the acidic buffer is citric acid buffer or acetic acid buffer; and/or
(e) the wash solution is trifluoroacetic acid solution or hydrochloric acid solution.

14. The method of any one of claims 1-11 wherein the target nucleic acid is Hepatitis B viral nucleic acid and the biological sample comprises Hepatitis B virus, and wherein the method further comprises heating the sample between steps (c) and (d).

15. The method of claim 14, wherein:
(a) the biological sample is heated to a temperature between 50ºC and 80ºC, optionally wherein the biological sample is heated to a temperature between 65ºC and 75ºC; and/or
(b) the step of heating is carried out for 30 seconds to 90 seconds, optionally wherein the step of heating is carried out for 50 seconds to 70 seconds.

## Patentansprüche

1. Verfahren zur Extraktion einer Zielnukleinsäure aus einer biologischen Probe umfassend Zellen oder Mikroorganismen, das Verfahren umfassend:
(a) Zugabe eines Übergangsmetallsalzes, eines Erdalkalimetallsalzes oder Kombination davon zu der biologischen Probe in einer Menge, die ausreichend ist, um Nukleasen in der Probe zu inaktivieren;
(b) Zugabe einer Extraktionslösung zu der biologischen Probe in einer Menge, die ausreichend ist, um die Zellen oder Mikroorganismen zu lysieren, wodurch ein Lysat entsteht;
(c) Zugabe eines sauren Puffers zu dem Lysat zu einer Menge, die ausreichend ist, um den pH des Lysats auf zwischen pH 1 bis pH 5 zu reduzieren; und
(d) Trennen der Zielnukleinsäure von dem Lysat, um die Zielnukleinsäure aus der biologischen Probe zu extrahieren.

2. Verfahren nach Anspruch 1, ferner umfassend die Durchführung einer Nukleinsäureamplifikation, gegebenenfalls unter Verwendung von PCR, nach Schritt (d) und den Nachweis des amplifizierten Nukleinsäureprodukts.

3. Verfahren nach einem der Ansprüche 1 bis 2, wobei die biologische Probe Vollblut, Serum, Plasma, Auswurf, Speichel, Urin, Stuhl oder Gewebe ist.

4. Verfahren nach einem der Ansprüche 1 bis 3, wobei die Zielnukleinsäure DNA, gegebenenfalls virale DNA, ist.

5. Verfahren nach einem der Ansprüche 1 bis 4, wobei die Extraktionslösung Proteinase K, Tensid oder eine Kombination davon umfasst.

6. Verfahren nach Anspruch 5, wobei das Tensid kationisches Tensid, gegebenenfalls Cetrimoniumbromid, ist.

7. Verfahren nach Anspruch 5, wobei das Tensid nicht ionisches Tensid, gegebenenfalls Triton X-100, ist.

8. Verfahren nach einem der Ansprüche 1 bis 7, wobei Schritt (d) durchgeführt wird durch Zugabe von magnetischen Partikeln zu dem Lysat in Schritt (c); Trennen der magnetischen Mikropartikel von dem Lysat unter Verwendung von magnetischer Mikropartikeltrennung; Zugabe einer Waschlösung zu den magnetischen Mikropartikeln und Zugabe eines Elutionspuffers zu den magnetischen Mikropartikeln, um die Nukleinsäure zu eluieren.

9. Verfahren zum Nachweis eines amplifizierten Nukleinsäureprodukts in einer Probe umfassend eine Zielnukleinsäure und Nukleasen, das Verfahren umfassend:
(a) Zugabe eines Übergangsmetallsalzes, eines Erdalkalimetallsalzes oder Kombination davon zu der Probe in einer Menge, die ausreichend ist, um die Nukleasen in der Probe zu inaktivieren;
(b) Zugabe eines sauren Puffers zu der Probe in einer Menge, die ausreichend ist, um den pH der Probe auf weniger als pH 5 zu reduzieren;
(c) Trennen der Zielnukleinsäure von der Probe;
(d) Durchführen einer Nukleinsäureamplifikationsreaktion, um das amplifizierte Nukleinsäureprodukt aus der Zielnukleinsäure herzustellen;
(e) Nachweisen des amplifizierten Nukleinsäureprodukts.

10. Verfahren nach Anspruch 9, wobei die Nukleinsäureamplifikationsreaktion ein PCR Prozess ist und/oder wobei die Zielnukleinsäure DNA, gegebenenfalls virale DNA, ist.

11. Verfahren nach einem der Ansprüche 1-10, wobei:
(a) Schritt (a) und Schritt (b) gleichzeitig durchgeführt werden; und/oder
(b) das Übergangsmetallsalz ausgewählt ist aus der Gruppe bestehend aus einem Mangansalz oder einem Zinksalz; und/oder
(c) das Erdalkalimetallsalz ausgewählt ist aus der Gruppe bestehend aus einem Magnesiumsalz oder einem Calciumsalz; und/oder
(d) der saure Puffer Zitronensäurepuffer oder Essigsäurepuffer ist; und/oder
(e) die Zielnukleinsäure RNA gegebenenfalls virale RNA ist.

12. Verwendung eines Kits zur Extraktion einer Nukleinsäure aus einer biologischen Probe umfassend Nukleinsäuren, Nukleasen und Inhibitoren umfassend:
(a) eine Extraktionslösung;
(b) eine Übergangsmetallsalzlösung, eine Erdalkalimetallsalzlösung oder Kombination davon;
(c) einen sauren Puffer und
(d) eine Waschlösung;
in einem Verfahren gemäß Anspruch 1.

13. Verwendung nach Anspruch 12, wobei
(a) die Extraktionslösung Proteinase K, ein Tensid oder eine Kombination davon umfasst und/oder
(b) das Übergangsmetallsalz ausgewählt ist aus der Gruppe bestehend aus einem Mangansalz oder einem Zinksalz und/oder
(c) das Erdalkalimetallsalz ausgewählt ist aus der Gruppe bestehend aus einem Magnesiumsalz oder einem Calciumsalz; und/oder
(d) der saure Puffer Zitronensäurepuffer oder Essigsäurepuffer ist; und/oder
(e) die Waschlösung Trifluoressigsäurelösung oder Salzsäurelösung ist.

14. Verfahren nach einem der Ansprüche 1-11 wobei die Zielnukleinsäure Hepatitis B virale Nukleinsäure ist und die biologische Probe Hepatitis B Virus umfasst und wobei das Verfahren ferner das Erhitzen der Probe zwischen den Schritten (c) und (d) umfasst.

15. Verfahren nach Anspruch 14, wobei:
(a) die biologische Probe auf eine Temperatur zwischen 50 °C und 80 °C erhitzt wird, gegebenenfalls wobei die biologische Probe auf eine Temperatur zwischen 65 °C und 75 °C erhitzt wird; und/oder
(b) der Schritt des Erhitzens für 30 Sekunden bis 90 Sekunden durchgeführt wird, gegebenenfalls wobei der Schritt des Erhitzens für 50 Sekunden bis 70 Sekunden durchgeführt wird.

## Revendications

1. Procédé d'extraction d'un acide nucléique cible à partir d'un échantillon biologique comprenant des cellules ou des micro-organismes, le procédé comprenant :
(a) l'ajout d'un sel de métal de transition, d'un sel de métal alcalino-terreux, ou d'une combinaison de ceux-ci, à l'échantillon biologique en une quantité suffisante pour inactiver les nucléases dans l'échantillon ;
(b) l'ajout d'une solution d'extraction à l'échantillon biologique en une quantité suffisante pour lyser les cellules ou les micro-organismes, en formant ainsi un lysat ;
(c) l'ajout d'un tampon acide au lysat en une quantité suffisante pour réduire le pH du lysat entre un pH 1 et un pH 5 ; et
(d) la séparation de l'acide nucléique cible du lysat pour extraire l'acide nucléique cible à partir de l'échantillon biologique.

2. Procédé selon la revendication 1, comprenant en outre la réalisation d'une amplification d'acide nucléique, facultativement en utilisant une PCR, après l'étape (d) et la détection du produit d'acide nucléique amplifié.

3. Procédé selon l'une quelconque des revendications 1 à 2, dans lequel l'échantillon biologique est du sang total, du sérum, du plasma, des expectorations, de la salive, de l'urine, des selles, ou un tissu.

4. Procédé selon l'une quelconque des revendications 1 à 3, dans lequel l'acide nucléique cible est de l'ADN, facultativement de l'ADN viral.

5. Procédé selon l'une quelconque des revendications 1 à 4, dans lequel la solution d'extraction comprend la protéinase K, un détergent, ou une combinaison de ceux-ci.

6. Procédé selon la revendication 5, dans lequel le détergent est un détergent cationique, facultativement le bromure de cétrimonium.

7. Procédé selon la revendication 5, dans lequel le détergent est un détergent non ionique, facultativement le Triton X-100.

8. Procédé selon l'une quelconque des revendications 1 à 7, dans lequel l'étape (d) est réalisée par l'ajout de particules magnétiques au lysat à l'étape (c) ; la séparation des microparticules magnétiques du lysat en utilisant une séparation de microparticules magnétiques ; l'ajout d'une solution de lavage aux microparticules magnétiques, et l'ajout d'un tampon d'élution aux microparticules magnétiques pour éluer l'acide nucléique.

9. Procédé de détection d'un produit d'acide nucléique amplifié dans un échantillon comprenant un acide nucléique cible et des nucléases, le procédé comprenant :
(a) l'ajout d'un sel de métal de transition, d'un sel de métal alcalino-terreux, ou d'une combinaison de ceux-ci, à l'échantillon en une quantité suffisante pour inactiver les nucléases dans l'échantillon ;
(b) l'ajout d'un tampon acide à l'échantillon en une quantité suffisante pour réduire le pH du lysat à moins d'un pH 5 ;
(c) la séparation de l'acide nucléique cible à partir de l'échantillon ;
(d) la réalisation d'une réaction d'amplification d'acide nucléique afin de produire le produit d'acide nucléique amplifié à partir de l'acide nucléique cible ;
(e) la détection du produit d'acide nucléique amplifié.

10. Procédé selon la revendication 9, dans lequel la réaction d'amplification d'acide nucléique est un procédé de PCR et/ou dans lequel l'acide nucléique cible est de l'ADN, facultativement de l'ADN viral.

11. Procédé selon l'une quelconque des revendications 1 à 10, dans lequel :
(a) l'étape (a) et l'étape (b) sont réalisées simultanément ; et/ou
(b) le sel de métal de transition est sélectionné dans le groupe consistant en un sel de manganèse ou un sel de zinc ; et/ou
(c) le sel de métal alcalino-terreux est sélectionné dans le groupe consistant en un sel de magnésium ou un sel de calcium ; et/ou
(d) le tampon acide est un tampon d'acide citrique ou un tampon d'acide acétique ; et/ou
(e) l'acide nucléique cible est de l'ARN, facultativement de l'ARN viral.

12. Utilisation d'un kit pour extraire un acide nucléique à partir d'un échantillon biologique comprenant des acides nucléiques, des nucléases et des inhibiteurs, comprenant :
(a) une solution d'extraction ;
(b) une solution de sel de métal de transition, une solution de sel de métal alcalino-terreux, ou une combinaison de celles-ci ;
(c) un tampon acide, et
(d) une solution de lavage ;
dans un procédé selon la revendication 1.

13. Utilisation selon la revendication 12, dans laquelle
(a) la solution d'extraction comprend la protéinase K, un détergent, ou une combinaison de ceux-ci et/ou
(b) le sel de métal de transition est sélectionné dans le groupe consistant en un sel de manganèse ou un sel de zinc et/ou
(c) le sel de métal alcalino-terreux est sélectionné dans le groupe consistant en un sel de magnésium ou un sel de calcium ; et/ou
(d) le tampon acide est un tampon d'acide citrique ou un tampon d'acide acétique ; et/ou
(e) la solution de lavage est une solution d'acide trifluoroacétique ou une solution d'acide chlorhydrique.

14. Procédé selon l'une quelconque des revendications 1 à 11, dans lequel l'acide nucléique cible est un acide nucléique viral de l'hépatite B et l'échantillon biologique comprend le virus de l'hépatite B, et où le procédé comprend en outre le chauffage de l'échantillon entre les étapes (c) et (d).

15. Procédé selon la revendication 14, dans lequel :
(a) l'échantillon biologique est chauffé à une température comprise entre 50 °C et 80 °C, facultativement dans lequel l'échantillon biologique est chauffé à une température comprise entre 65 °C et 75 °C ; et/ou
(b) l'étape de chauffage est réalisée pendant 30 secondes à 90 secondes, facultativement dans lequel l'étape de chauffage est réalisée pendant 50 secondes à 70 secondes.
